# EUROPÄISCHE PATENTSCHRIFT

(11) **EP 2 663 773 B1**
(45) Veröffentlichungstag und Bekanntmachung des Hinweises auf die Patenterteilung: **11.01.2017**
(21) Anmeldenummer: 12780766.7
(22) Anmeldetag: 06.11.2012
(51) Int. Cl.: F04B 13/00, F04B 19/02, F04B 43/08, G01F 1/05, G01F 1/37

(54) **VORRICHTUNG ZUR ZUFÜHRUNG UND DOSIERUNG EINES FLUIDS FÜR MEDIZINISCHE ZWECKE**
DEVICE FOR SUPPLYING AND METERING A FLUID FOR MEDICINAL PURPOSES
DISPOSITIF SERVANT À AMENER ET À DOSER UN FLUIDE À DES FINS MÉDICALES

(30) Priorität: 19.03.2012 DE 102012102273
(43) Veröffentlichungstag der Anmeldung: 20.11.2013
(73) Patentinhaber: B. Braun Melsungen AG, 34212 Melsungen (DE)
(72) Erfinder: EBERHARD, Dietmar, 79341 Kenzingen (DE)
(74) Vertreter: Winter, Brandl, Fürniss, Hübner, Röss, Kaiser, Polte - Partnerschaft mbB
(86) Internationale Anmeldenummer: PCT/EP2012/071942
(87) Internationale Veröffentlichungsnummer: WO 2013/139408

(56) Entgegenhaltungen:
- DE-A1- 3 209 721
- DE-C1- 3 838 689
- US-A- 2 274 479
- US-A- 3 178 942
- US-A- 3 802 265
- US-B1- 6 171 253

## Beschreibung

Die Erfindung betrifft eine Vorrichtung zur Zuführung und Dosierung eines Fluids für medizinische Zwecke, wenigstens umfassend eine Pumpe zur Förderung des Fluids und ein Bauteil, durch den das Fluid gefördert wird.

Eine solche Vorrichtung wird insbesondere in der Infusions- oder Dialysetechnik eingesetzt, um ein Fluid zu fördern und einem Patienten dosiert zuzuführen, wobei in diesem Bereich vorwiegend Peristaltik- und Spritzenpumpen zum Einsatz kommen. Doch auch Kolbenpumpen können vorteilhaft verwendet werden, wie es beispielsweise die US 7,887,308 B2 offenbart. Mittels einer Kolbenpumpe ist es möglich, auch bei großen Fördervolumen eine hohe Fördergenauigkeit für ein Fluid zu erreichen, das aus einem austauschbaren Vorratsbehälter angesaugt wird.

Oftmals ist es dabei erstrebenswert, eine Pumpe und andere Komponenten sehr kompakt in ein Gehäuse integrieren zu können. Dies ist beispielsweise der Fall, wenn es sich um medizinische Einmalartikel und/oder Infusionstechnik im Bereich der häuslichen Pflege handelt, wobei Pumpen für austauschbare Infusionsbehälter einfach vom Patienten, ungelernten Angehörigen oder dem Pflegepersonal anzuschließen sein sollen. Hierbei hat es sich als vorteilhaft erwiesen, die gesamte Pumpe mit ihren zusätzlichen Komponenten in einer Art Kassette unterzubringen. Zu diesen zusätzlichen Komponenten zählen insbesondere Sensoriken zur Feststellung von Okklusionen, um gefährliche Verstopfungen oder sogar dem Verschluss von Leitungen zu erkennen.

Die Erkennung einer Okklusion erfolgt meist durch eine indirekte Messung des Innendrucks in einem Schlauch, der zur Zuführung einer Flüssigkeit zu einem Patienten dient. Besteht eine Okklusion, erhöht sich beispielsweise stromab der Pumpe der Innendruck im Schlauch, was indirekt gemessen werden kann. Hierzu wird der runde Schlauchquerschnitt beispielsweise durch eine Vorspannkraft elliptisch verformt und der zu bestimmende Innendruck des Schlauchs erhöht oder vermindert diese Vorspannkraft, die dann mittels eines Kraftsensors ermittelt werden kann. Die DE 38 38 689 C1 offenbart exemplarisch ein derartiges Verfahren zur Druckmessung und Okklusionserkennung.

Beim Einlegen eines Schlauchsets in eine Pumpe muss nach heutigem Stand der Technik daher oftmals das für eine Okklusionssensorik zuständige Schlauchsegment zusätzlich per Hand in spezielle Halterungen eingelegt werden, was nicht nur im Bereich der häuslichen Pflege problematisch sein kann. Nachteilig bei dieser Methode ist ferner, dass die Verformung des Schlauchs in der Regel zu einem über Stunden anhaltenden Kriechvorgang führt. Dieses Kriechen baut Spannungen im Schlauchquerschnitt ab, was zu einer stetigen Änderung der gemessenen Kraft führt. Die durch das Kriechen verursachte und unerwünschte Kraftänderung ist in einer ähnlichen Größenordnung wie der gewünschte Messeffekt durch die Variation des Schlauchinnendrucks und erschwert somit die sichere Erkennung einer Okklusion. Spezielle Elastomere wie beispielsweise Silikone weisen ein deutlich vermindertes Kriechverhalten auf und sind daher als Schlauchsegment für die Okklusionssensorik prädestiniert. Eine Verbindung von Silikon- mit Nichtsilikonmaterialien ist allerdings sehr aufwändig, da prozesssichere Klebungen nicht möglich sind.

Ferner sind zur Druckmessung in der Medizintechnik sogenannte "Dome" oder "Druck-dome" bekannt. Diese weisen typischerweise eine von einem Fluid durchströmbare Messkammer auf, wobei ein Teil der Wandung der Messkammer durch eine Membran gebildet ist, die wesentlich nachgiebiger ist als der übrige Teil der Wandung der Messkammer. Üblicherweise liegt an der Außenseite der Membran ein Messwertaufnehmer an, der mittels eines druckübertragenden Kontakts Bewegungen der Membran registriert. Ein Messwandler wandelt dann die über die Membran des Druckdomes übertragenen Drücke und Druckänderungen in ein elektrisches Signal um. Beispielsweise offenbart die EP 0 208 955 A2 hierzu ein Verfahren zur Herstellung eines druckübertragenden Kontakts zwischen der Membran eines Druckdomes und dem Druckübertrager eines Messwandlers.

Darüber hinaus offenbart die DE 43 40 536 A1 eine Vorrichtung zur Überwachung einer intravenösen Infusionstherapie, bei der Infusionsunterbrechungen aufgrund eines resultierenden Druckanstiegs im Infusionsschlauchsystem detektiert werden sollen. Dabei wird in das Schlauchsystem eine Druckübertragungskapsel mit einer Druckübertragungsmembran eingesetzt, die wiederum über einen flüssigkeitsgefüllten Spalt in Kontakt mit der Membran eines Drucksensors steht. So sollen sich auch kleine Druckanstiege rasch erkennen lassen, wenn sich die Druckübertragungsmembran aufgrund eines Druckanstiegs nach außen wölbt.

Auch andere Sensoriken, welche Druckänderungen direkt an einem Schlauch messen, der von einem Fluid durchflossen wird, sind bekannt. Beispielsweise können Schläuche dazu punktuell durch einen Messwertgeber verformt werden, der auf Druckänderungen an der Kontaktstelle reagiert, oder es befindet sich ein Messwertstreifen am Umfang des Schlauches, der Umfangsänderungen aufgrund von Veränderungen des Innendrucks registriert. Ferner offenbart beispielsweise die DE 30 35 703 A1 ein Infusionsgerät mit einem Drucksensor zur Erkennung von Okklusionen, bei dem der Schlauch zwischen einem festen und einem gefederten/elastischen Bauteil eingeklemmt wird, so dass Druckänderungen im Schlauch indirekt über die Deformierung des elastischen Bauteils detektiert werden können.

Die US 3,802,265 offenbart eine Vorrichtung zur Messung der Fliessgeschwindigkeit eines Fluids in einem Durchgang.

Die Vorrichtung arbeitet mit einer Druckwaage, wobei eine elastische Membran den Fluidstömungsraum von einer Messdruckkammer separiert.

Die US 3 178 942 A zeigt eine Messvorrichtung zur Strömungsbestimmung. Die Messvorrichtung umfasst ein Durchflussmessergehäuse mit einem Fluiddurchgang mit einem oberen Abschnitt und einer Drossel von kleinerem Durchmesser, so dass sich ein Druckunterschied gegenüber dem oberen Abschnitt ergibt. Der Fluiddruck wird durch eine Öffnung gegenüber dem Durchgang gemessen, in welcher sich ein Druckaufnehmer befindet.

Die Messung erfolgt über eine hydraulische Druckwaage (Differenzdruckmessgerät).

US 2,274,479 folgt dem Konstruktionsprinzip der Ausbildung einer Messdruckkammer für den hydraulischen Abgriff des Innendrucks in einem Fluidströmungsraum.

Ein erstes Element mit einer "weichen" Wandung wird von Fluid durchströmt. Um dieses Element herum ist ein zweites Element gebaut, welches eine "harte" Wandung aufweist. Zwischen den Elementen ist ein Hohlraum ausgebildet, der mit einer inkompressiblen Flüssigkeit gefüllt wird. Druckunterschiede eines Fluids, welches durch das Element mit der "weichen" Wand strömt, macht sich in Änderungen der Ausdehnung dieses Elements bemerkbar. Da sich das zweite Element mit der "harten" Wandung nicht verändert, kann in der inkompressiblen Flüssigkeit zwischen dem ersten und dem zweiten Element ein Druckverhalten gemessen werden, welches den Druckverlauf im Fluid durch das erste Element repräsentiert.

Die DE 32 09 721 A1 zeigt eine Drucküberwachungseinrichtung, bei der ein Flüssigkeits- oder Deformationsmanometer benutzt wird, bei dem die Deformation, bedingt durch Druckanstieg, einen Schalter betätigt.

Die US 6 171 253 zeigt eine Messeinrichtung mit Dehnungsmesselementen.

Die Messelemente nehmen die Längenausdehnung einer Membran auf, die durch Flüssigkeitsdruck gedehnt wird. Das Messergebnis wird direkt elektrisch an den Dehnungsmesselementen abgenommen.

Alle bekannten Vorgehensweisen sind jedoch relativ aufwändig bzw. störanfällig und eignen sich nur bedingt für eine kompakte Anordnung in einem Gehäuse. Aufgabe der Erfindung ist es daher, eine Vorrichtung zur Zuführung und Dosierung eines Fluids für medizinische Zwecke bereitzustellen, welche eine solche kompakte Bauweise und einfache Handhabung ermöglicht.

Erfindungsgemäß wird diese Aufgabe durch eine Vorrichtung gemäß dem unabhängigen Anspruch 1 gelöst. Vorteilhafte Weiterbildungen dieser Vorrichtung ergeben sich aus den Unteransprüchen 2-13.

Dabei umfasst die erfindungsgemäße Vorrichtung zur Zuführung und Dosierung eines Fluids für medizinische Zwecke wenigstens eine Pumpe zur Förderung des Fluids und ein Bauteil, durch den das Fluid gefördert wird. In dem Bauteil ist wenigstens eine Aussparung vorgesehen, welche dicht durch eine Sensorkomponente abgedeckt wird, die aus einem druckempfindlichen Material besteht. Dabei ist das Material des Bauteils härter als das der Sensorkomponente. Ferner weist die Vorrichtung einen Kraftsensor auf, mit dem druckinduzierte Veränderungen der Sensorkomponente im Bereich der Aussparung messbar sind. Die Erfindung sieht eine Okklusionssensorik somit in einem Bauteil einer Pumpe vor, durch welches das Fluid ohnehin gefördert wird, so dass keine separate Sensorik montiert werden muss.

Das Bauteil kann beispielsweise ein rohrförmiger Stutzen sein, durch den das Fluid zu der Pumpe hin oder von der Pumpe weg gefördert wird. Es handelt sich dann um einen Einlass- und/oder Auslassstutzen, der ein Gehäuse für die Sensorkomponente bildet und eine Aussparung aufweist, in welche die Sensorkomponente eingebracht ist. Das Bauteil kann jedoch auch ein ebener Flansch sein, an dem Stutzen und/oder Zylinder zur Förderung des Fluids angebracht sind, wobei das Fluid wenigstens teilweise entlang des Flansches strömt. Der Flansch bildet dann das harte Gehäuse für die weiche Sensorkomponente. Sensorkomponenten können auch sowohl in einen Flansch, als auch in einen oder mehreren Stutzen eingebracht werden.

Die Erfindung macht sich somit das Funktionsprinzip einer Druckmembran zunutze, setzt dieses jedoch nicht in einem separaten Bauteil ein, sondern integriert eine entsprechende Sensorkomponente in ein Bauteil aus einem harten Material, durch den ein Fluid ohnehin gefördert wird. Dabei basiert die zu integrierende mechanische Sensorkomponente der Okklusionssensorik auf dem Prinzip der Druckmessung im Fluid und wird durch ein elastischen Material realisiert, das sich physikalisch analog einer Druckmembran verhält. Das jeweilige Bauteil, welches als Gehäuse genutzt wird, bildet eine druckunempfindliche Hartkomponente, die sich bei den auftretenden Druckänderungen nicht verformt. Aufgrund der auftretenden Verformungen der Sensorkomponente als Weichkomponente kann dagegen auf den Innendruck in diesem Bauteil geschlossen werden.

Die Okklusionssensorik kann so platzsparend direkt in ein hartes Bauteil integriert werden, was eine sehr kompakte Bauweise ermöglicht. Bei dem Bauteil handelt es sich insbesondere um einen Einlass- und/oder Auslassstutzen, der das Fluid zu einer Pumpe bzw. von dieser zu einem Patienten führt. Die Sensorik kann so Okklusionen vor und/oder hinter einer Pumpe erkennen. Wird der zugehörige Stutzen entsprechend so positioniert, dass er kompakt mit einer Pumpe in einem Gehäuse untergebracht werden kann, wird durch die Okklusionssensorik an diesem Stutzen kaum weiterer Bauraum benötigt. Insbesondere handelt es sich bei der eingesetzten Pumpe um eine volumetrische Kolbenpumpe.

Die Sensorkomponente ist dabei vorzugsweise ein integraler und nicht demontierbarer Bestandteil des betreffenden Bauteils, so dass sie auch nicht bei Inbetriebnahme der Vorrichtung installiert oder sogar ausgerichtet werden muss. Dies erleichtert die Handhabung der Vorrichtung und vermeidet Einrichtungsfehler und somit auch Messfehler.

Der Kraftsensor hat im Bereich der Aussparung Kontakt mit der Oberfläche der Sensorkomponente, wobei der Kraftsensor einen Stempel aufweist, der im Bereich der Aussparung in direktem Kontakt mit der Oberfläche der Sensorkomponente steht. So kann eine Veränderung der Ausdehnung der Sensorkomponente in diesem Bereich gemessen werden.

Vorzugsweise besteht die Sensorkomponente aus einem Elastomer, wobei es sich insbesondere um Silikon oder ein thermoelastisches Elastomer handeln kann. So können die physikalischen Eigenschaften dieser speziellen Elastomere vorteilhaft genutzt werden, was insbesondere ein geringes Kriechverhalten beinhaltet. Eine stoffschlüssige Verbindung von Silikon- und Nichtsilikonmaterialien ist dabei jedoch nicht erforderlich, da für eine dichte Verbindung zwischen Stutzen und Sensorkomponente geeignete Verfahren wie beispielsweise Spritzgussverfahren eingesetzt werden können. So können der Stutzen und die Sensorkomponente in einem Zweikomponentenverfahren hergestellt werden. Alternativ kann die Verbindung zwischen Stutzen und Sensorkomponente auch durch andere Verbindungstechniken hergestellt werden, wobei beispielsweise Steck-, Klick-, Schraub- oder auch Klebeverbindungen in Frage kommen.

In einem ersten Ausführungsbeispiel der Erfindung ist die Sensorkomponente ein Schlauch, der einen Stutzen formschlüssig umgibt, so dass sie die Aussparung dicht von außen abdeckt. In einem anderen Ausführungsbeispiel ist dieser Schlauch formschlüssig innerhalb eines Stutzens angebracht, so dass die Sensorkomponente die Aussparung dicht von innen abdeckt. Dabei haben der Stutzen und die Sensorkomponente zweckmäßigerweise einen ähnlichen oder gleichen Querschnitt. Beispielsweise kann ein Schlauch mit rundem Querschnitt formschlüssig in einen runden Stutzen eingebracht werden bzw. diesen umschließen.

Allerdings kann es auch vorteilhaft sein, wenn die Sensorkomponente einen elliptischen Querschnitt hat, wobei eine flache Seite der Sensorkomponente im Bereich der Aussparung angeordnet ist. Dies kann sowohl für innen als auch für außen liegende Sensorkomponenten der Fall sein, wobei der Querschnitt des Stutzens entsprechend angepasst werden muss. Durch diese Form der Weichkomponente hat die Sensorkomponente bereits die für die Innendruckmessung notwendige elliptische Verformung, so dass unerwünschtes Kriechverhalten bei thermoplastischen Elastomeren bereits weitestgehend unterbunden werden kann.

Der elliptische Querschnitt kann beispielsweise dadurch erreicht werden, dass ein Schlauch mit ursprünglich rundem Querschnitt entsprechend verformt wird, bevor er an einem Ein- oder Auslassstutzen montiert wird. Die Verformung wird dann nicht durch die Montage bewirkt, sondern um unerwünschtes Kriechverhalten zu unterbinden, erfolgt eine Vorverformung des Schlauches auf den gewünschten elliptischen Querschnitt.

In einem anderen Ausführungsbeispiel der Erfindung ist die Sensorkomponente eine speziell geformte Messmembran mit einem Querschnitt, der wenigstens zwei gegenüber liegende Membranseiten aufweist, die jeweils nach innen geknickt sind, während eine Membranoberseite, welche die beiden Membranseiten miteinander verbindet, gerade ausgeformt und im Bereich der Aussparung angeordnet ist. Der Kraftsensor liegt somit an einer geraden Fläche der Messmembran an, die nicht mehr durch die Eigenspannung verändert wird, so dass sich eine lineare Kraftkennlinie ergibt.

Mögliche Einsatzgebiete der erfindungsgemäßen Vorrichtung mit Pumpe sind u.a. medizinischen Einmalartikel von Infusions- oder Dialysesystemen oder Geräte mit Einmalartikeln zur individuellen Dosierung von Medikamenten z.B. im Apothekenbereich. Die Vorrichtung kann insbesondere in ein medizinisches Infusions-Set integriert werden und schafft so die Voraussetzung für ein kompaktes Kassettensystem für eine Pump- und Sensoreinheit. Die Erfindung ist jedoch nicht auf Infusions-Sets beschränkt, sondern kann für alle medizinischen Anwendungsgebiete genutzt werden, bei denen ihre Eigenschaften vorteilhaft sind.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:
- Fig. 1: einen Längsschnitt durch einen Stutzen mit außen liegender Sensorkomponente;
- Fig. 2: einen Querschnitt durch einen Stutzen gemäß Fig. 1;
- Fig. 3: einen Längsschnitt durch einen Stutzen mit einem ersten Ausführungsbeispiel einer innen liegenden Sensorkomponente;
- Fig. 4: einen ersten Querschnitt durch einen Stutzen gemäß Fig. 3;
- Fig. 5: einen zweiten Querschnitt durch einen Stutzen gemäß Fig. 3;
- Fig. 6: einen Querschnitt durch einen Stutzen mit einem zweiten Ausführungsbeispiel einer innen liegenden Sensorkomponente; und
- Fig. 7: eine Sensorkomponente an einem Pumpenflansch.

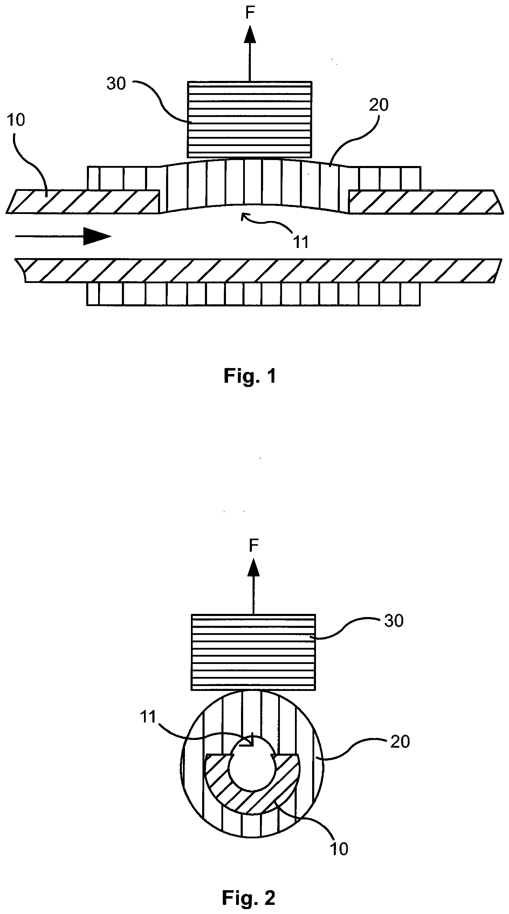

Der in Fig. 1 dargestellte Längsschnitt durch einen Stutzen 10 zeigt eine außen liegende Sensorkomponente 20, welche den Stutzen 10 im Bereich einer Aussparung 11 formschlüssig umgibt. Hierbei wird eine dichte Verbindung zwischen dem Stutzen 10 und der schlauchförmigen Sensorkomponente 20 erreicht. Die Sensorkomponente 20 kann dabei an ihrer Innenseite so ausgeformt sein, dass sie sich teilweise in die Aussparung 11 einfügt, wie es in Fig. 1 dargestellt ist.

Die Aussparung 11 kann einen beliebigen Querschnitt haben, wobei sich runde Querschnitte für eine gleichmäßige Kraftverteilung als vorteilhaft erwiesen haben. Ferner sollte die Größe der Aussparung 11 geeignet gewählt werden. In Fig. 2 ist beispielsweise ein Querschnitt durch die Mitte des Längsschnitts aus Fig. 1 gezeigt, bei dem die Aussparung 11 sehr tief gewählt wurde und annähernd bis zur Mittellinie des Stutzens 10 reicht.

Durch die Aussparung 11 kann dann ein Kraftsensor reichen, um in diesem Bereich Kontakt mit der Außenseite der Sensorkomponente 20 herzustellen. Dies kann beispielsweise über einen Stempel 30 erfolgen, der an der Sensorkomponente anliegt. Erhöht sich der Innendruck im Stutzen 10 aufgrund einer Okklusion, wölbt sich die Sensorkomponente 20 nach außen, was von dem Stempel 30 detektiert werden kann.

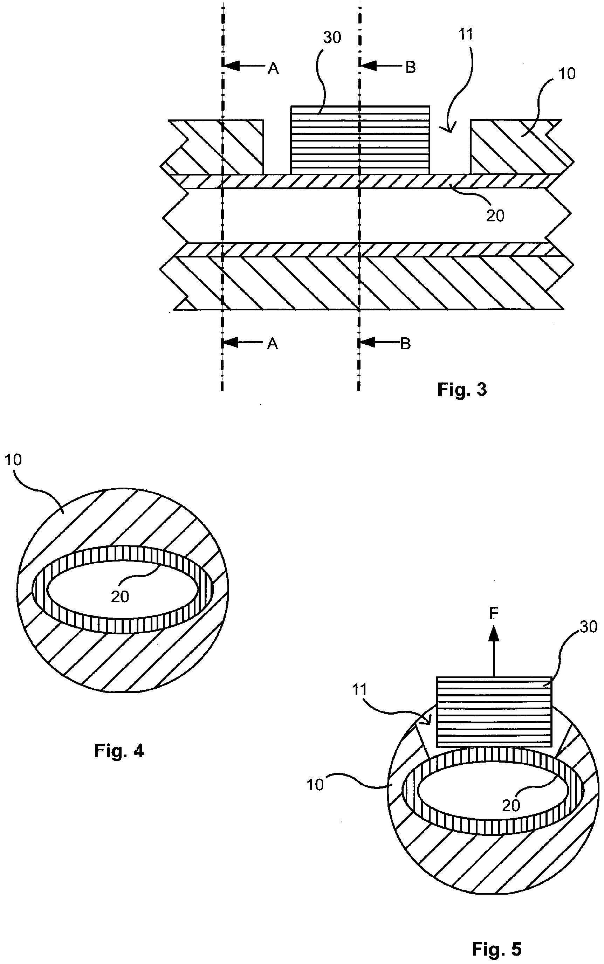

Fig. 3 zeigt ein zweites Ausführungsbeispiel der Erfindung, bei dem eine schlauchförmige Sensorkomponente 20 innerhalb eines Stutzens 10 angebracht ist und somit von innen eine Aussparung 11 dicht abdeckt. Der Stempel 30 eines Kraftsensors kann dann durch die Aussparung 11 hindurch die Sensorkomponente 20 kontaktieren. Dabei kann die Innenfläche des Stutzens 10 auch so ausgebildet sein, dass sie den Schlauch 20 in seiner Position hält und an einem axialen Verrutschen hindert (nicht dargestellt).

Fig. 4 zeigt einen ersten Querschnitt durch einen solchen Stutzen entlang der Linie A-A, wodurch ersichtlich wird, dass die Sensorkomponente 20 einen elliptischen Querschnitt hat. Die Innenwandung des Stutzens 10 ist entsprechend ausgeformt, um die Sensorkomponente 20 formschlüssig aufnehmen zu können. Ein zweiter Querschnitt entlang der Linie B-B ist in Fig. 5 dargestellt und zeigt den Stempel 30, der durch die Aussparung 11 hindurch die Außenfläche der Sensorkomponente 20 kontaktiert.

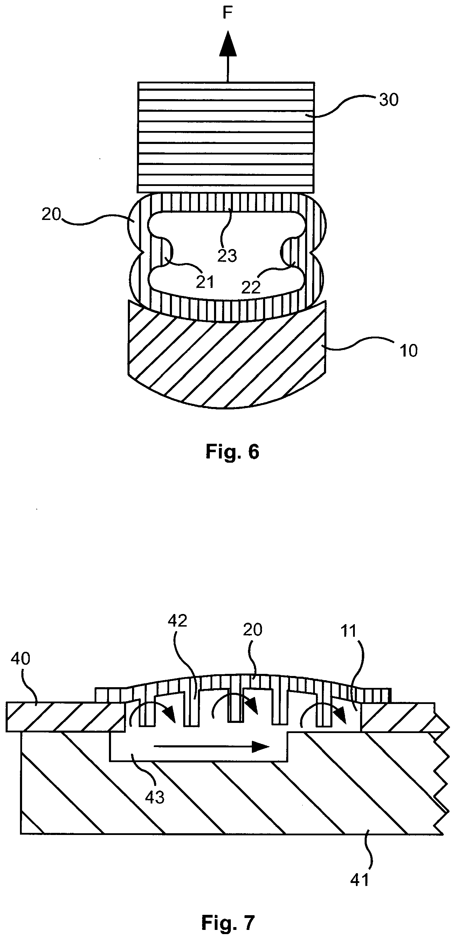

Um Eigenspannungen der Sensorkomponente 20 weitestgehend zu verhindern, kann diese auch als speziell geformte Messmembran ausgebildet sein, wie sie beispielsweise in Fig. 6 dargestellt ist. Hierbei weist die Messmembran 20 zwei gegenüber liegende Membranseiten 21 und 22 auf, die nach innen geknickt sind. Die Membranoberseite 23, welche die beiden Membranseiten 21, 22 verbindet, ist gerade ausgeführt und steht in Kontakt mit dem Stempel 30. Die Membranoberseite 23 verändert sich so nicht mehr durch die Eigenspannung, was eine lineare Kraftkennlinie Kraft = Innendruck x Membranfläche ergibt.

Der Querschnitt der Sensorkomponente 20 ist somit individuell gestaltet und enthält wenigstens eine der folgenden funktionalen Komponenten:
Eine gerade oder einer Geraden ähnliche Linie, welche die Geometrie der für die Messzwecke benötigte Membran bestimmt
Eine gerade oder gebogene Linie gegenüber der Membran, die eine Stützfunktion der Weichkomponente gegenüber der rohr- oder skelettförmigen Hartkomponente wahrnimmt.
Eine Geometrie zur Realisierung einer federnden Funktion an den beiden Seiten der Weichkomponente, damit eine Vorspannung aufgebaut werden kann, die zur Messung von Drücken unterhalb des atmosphärischen Umgebungsdrucks notwendig ist. Darüber hinaus ist die federnde Funktion notwendig, dass sich die Membran bei einem steigenden Innendruck von ihrer gegenüberliegenden Stützfläche entfernen kann.

Die Innenfläche des Stutzens 10 kann dann entsprechend ausgeführt sein, so dass die Messmembran 20 formschlüssig an diesem anliegt und sich die Messmembran bei einer Druckerhöhung nicht in unerwünschte Richtungen z.B. zur Seite hin ausdehnt. Diese spezielle Ausformung des Stutzens 10 kann auch nur im Bereich der Okklusionssensorik gegeben sein, wodurch sich aufwändige Formen innerhalb des gesamten Stutzens vermeiden lassen.

Die Hartkomponente, die den Stempel 30 umgibt, weist vorzugsweise eine ebene Fläche auf, die etwas unterhalb der Stempeloberkante liegt. Diese Fläche dient als Anschlagsfläche, wenn der Stempel gegen eine andere Fläche gedrückt wird. Der Stempel kann dann nur um den Betrag seines Überstandes eingedrückt werden, wodurch eine konstante Vorspannung für die Drucksensorik erzeugt wird.

Fig. 7 zeigt eine Ausführungsform der Erfindung, bei der die Okklusionssensorik direkt auf einem ebenen Flansch 40 angebracht ist, der Teil einer Pumpe ist. An diesem Flansch 40 können Stutzen und/oder Zylinder der Pumpe angebracht sein, es kann jedoch auch eine Ventilscheibe 41 von unten an dem Flansch 40 anliegen. Bei der Förderung strömt ein Fluid durch diese Ventilscheibe und dabei wenigstens teilweise entlang des Flansches 40, wobei in der Ventilscheibe 41 eine Vertiefung 43 vorgesehen ist, durch welche das Fluid dabei strömt. Innerhalb des Flansches 40 ist eine Aussparung 11 vorgesehen, welche von einer Sensorkomponente 20 abgedeckt wird. Um eine größere Fläche und damit eine erhöhte Druckempfindlichkeit bereitstellen zu können, ist die jeweilige Membran der Sensorkomponente 20 vorzugsweise deutlich breiter als die Breite der auf der Ventilscheibe 40 untergebrachten Vertiefung 43.

Die Hauptströmung des Fluids ist dabei durch einen horizontalen Pfeil nach rechts dargestellt. Möglicherweise kann das Volumen unterhalb der Membran 20 durch diese Hauptströmung jedoch nicht vollständig entlüftet wird. Die Funktion der Okklusionssensorik ist jedoch auch für diesen Fall vollständig gegeben. Durch eine zusätzliche Flüssigkeitsmenge, die benötigt wird, die Luft zu komprimieren, verzögert sich die Ansprechzeit bei einem nicht vollständig entlüfteten Volumen unterhalb der Membran 20 etwas. Wird die Membranunterseite mit einem einfach oder doppelt spiralförmigen Labyrinth 42 versehen, wird bei entsprechender Ausgestaltung der Kapillareffekt eine zusätzliche Anfangsströmung induzieren, die eine weitest gehende Entlüftung bewirken kann. Diese Anfangsströmung ist in Fig. 7 durch mehrere gebogene Pfeile im Uhrzeigersinn dargestellt.

Die von der Membran der Sensorkomponente 20 ausgehende Kraft wird auf einen externen Kraftsensor gegeben, um eine Okklusion zu erkennen (nicht dargestellt). Da eine Okklusion in einer Pumpenzuleitung beim Ansaugen der Pumpe einen Unterdruck zur Folge hat, müssen Membranen 20 im Bereich der Pumpenzuleitung durch ihr Design bereits eine Wölbung aufweisen, die sich dann durch den Unterdruck verringert.

### Bezugszeichenliste

- 10: Stutzen, Einlassstutzen, Auslassstutzen
- 11: Aussparung
- 20: Sensorkomponente, Membran
- 21,22: Membranseite
- 23: Membranoberseite
- 30: Kraftsensor, Stempel
- 40: Flansch
- 41: Ventilscheibe
- 42: Labyrinth
- 43: Vertiefung

## Patentansprüche

1. Vorrichtung zur Zuführung und Dosierung eines Fluids für medizinische Zwecke, umfassend eine Pumpe zur Förderung des Fluids und wenigstens ein Bauteil (10;40), durch welches das Fluid gefördert wird, wobei
in dem Bauteil (10;40) wenigstens eine Aussparung (11) vorgesehen ist, welche dicht durch eine Sensorkomponente (20) abgedeckt wird, die aus einem druckempfindlichen Material besteht, wobei das Material des Bauteils (10;40) härter ist als das der Sensorkomponente (20), und wobei die Vorrichtung einen Kraftsensor (30) aufweist, mit dem druckinduzierte Veränderungen der Sensorkomponente (20) im Bereich der Aussparung (11) messbar sind, **dadurch gekennzeichnet, dass** der Kraftsensor (30) einen Stempel aufweist, der im Bereich der Aussparung (11) in direktem Kontakt mit der Oberfläche der Sensorkomponente (20) steht, um eine Wölbung der Sensorkomponente (20) nach außen zu detektieren und auf den Kraftsensor (30) zu übertragen.

2. Vorrichtung nach Anspruch 1,
**dadurch gekennzeichnet, dass** das Bauteil ein rohrförmiger Stutzen (10) ist, durch den das Fluid zu der Pumpe hin oder von der Pumpe weg gefördert wird.

3. Vorrichtung nach einem oder beiden der Ansprüche 1 und 2,
**dadurch gekennzeichnet, dass** das Bauteil ein Flansch (40) ist, an dem rohrförmige Stutzen und/oder Zylinder zur Förderung des Fluids angebracht sind, wobei das Fluid wenigstens teilweise entlang des Flansches (40) strömt.

4. Vorrichtung nach einem oder mehreren der Ansprüche 1 bis 3,
**dadurch gekennzeichnet, dass** die Sensorkomponente (20) aus einem Elastomer besteht.

5. Vorrichtung nach Anspruch 4,
**dadurch gekennzeichnet, dass** die Sensorkomponente (20) aus Silikon oder einem thermoelastischen Elastomer besteht.

6. Vorrichtung nach einem oder mehreren der Ansprüche 1 bis 5,
**dadurch gekennzeichnet, dass** die Verbindung zwischen der Sensorkomponente (20) und dem Bauteil (10;40) eine Spritzgussverbindung ist, die durch ein Zweikomponentenverfahren hergestellt wurde.

7. Vorrichtung nach einem oder mehreren der Ansprüche 2 bis 6,
**dadurch gekennzeichnet, dass** die Sensorkomponente (20) ein Schlauch ist, der einen rohrförmigen Stutzen (10) formschlüssig umgibt, so dass sie die Aussparung (11) dicht von außen abdeckt.

8. Vorrichtung nach einem oder mehreren der Ansprüche 2 bis 6,
**dadurch gekennzeichnet, dass** die Sensorkomponente (20) ein Schlauch ist, der formschlüssig innerhalb eines rohrförmigen Stutzens (10) angebracht ist, so dass sie die Aussparung (11) dicht von innen abdeckt.

9. Vorrichtung nach einem oder beiden der Ansprüche 7 und 8,
**dadurch gekennzeichnet, dass** die Sensorkomponente (20) einen elliptischen Querschnitt hat, wobei eine flache Seite der Sensorkomponente (20) im Bereich der Aussparung (11) angeordnet ist.

10. Vorrichtung nach Anspruch 8,
**dadurch gekennzeichnet, dass** die Sensorkomponente (20) eine Messmembran mit einem Querschnitt ist, der wenigstens zwei gegenüber liegende Membranseiten (21 ;22) aufweist, die jeweils nach innen geknickt sind, während eine Membranoberseite (23), welche die beiden Membranseiten (21 ;22) miteinander verbindet, gerade ausgeformt und im Bereich der Aussparung (11) angeordnet ist.

11. Vorrichtung nach einem der vorstehenden Ansprüche,
**dadurch gekennzeichnet, dass** das Bauteil (10; 40) einen ebenen Flansch (40) und eine Ventilscheibe (41) mit einer Vertiefung (43) umfasst, wobei das Fluid wenigstens teilweise entlang des Flansches (40) durch die Vertiefung (43) strömt, und die Sensorkomponente (20) gegenüber der Vertiefung (43) ein spiralförmiges Labyrinth (42) aufweist zum Induzieren einer Anfangsströmung.

12. Vorrichtung nach einem der vorstehenden Ansprüche, **dadurch gekennzeichnet, dass** der Stempel von einer Hartkomponente umgeben ist, die unterhalb einer Stempeloberkante eine Anschlagsfläche aufweist, wobei die Anschlagsfläche dazu angeordnet ist, damit der Stempel nur um den Betrag seines Überstandes eingedrückt werden kann und/oder eine Vorspannung für eine Drucksensorik zu erzeugen.

13. Vorrichtung nach einem der vorstehenden Ansprüche, **dadurch gekennzeichnet, dass** die Sensorkomponente (20) eine Membran ist, wobei die von der Membran der Sensorkomponente (20) ausgehende Kraft auf den externen Kraftsensor (30) gegeben wird, um eine Okklusion zu erkennen.

## Claims

1. Apparatus for supplying and metering a fluid for medical purposes, including a pump for pumping the fluid and at least one element (10;40) through which the fluid is delivered, wherein
in the element (10;40) at least one recess (11) is provided which is tightly covered by a sensor component (20) composed of a pressure sensitive material, wherein the material of the element (10;40) is harder than that of the sensor component (20), and that the apparatus comprises a force sensor (30) with which pressure-induced changes of the sensor component (20) in the region of the recess (11) can be measured, **characterized in that** the force sensor (30) comprises a plunger which is in direct contact in the region of the recess (11) with the surface of the sensor component (20) in order to recognize an outward curvature of the sensor component (20) and to transfer it to the force sensor (30).

2. Apparatus according to claim 1,
**characterized in that** the element is a tubular port (10) through which the fluid is pumped toward or away from the pump.

3. Apparatus according to one or both of claims 1 and 2,
**characterized in that** the element is a flange (40) to which tubular ports and/or cylinders for pumping the fluid are attached, wherein the fluid flows at least partially along the flange (40).

4. Apparatus according to one or more of claims 1 to 3,
**characterized in that** the sensor component (20) is composed of an elastomer.

5. Apparatus according to claim 4,
**characterized in that** the sensor component (20) is composed of silicon or a thermoelastic elastomer.

6. Apparatus according to one or more of claims 1 to 5,
**characterized in that** the connection between the sensor component (20) and the element (10;40) is an injection-molded connection made by a two-component process.

7. Apparatus according to one or more of claims 2 to 6
**characterized in that** the sensor component (20) is a tube which surrounds a tubular port (10) with form-locking fit such that it tightly covers the recess (11) from the outside.

8. Apparatus according to one or more of claims 2 to 6,
**characterized in that** the sensor component (20) is a tube which is attached with form-locking fit inside a tubular port (10) such that it tightly covers the recess (11) from inside.

9. Apparatus according to one or both of claims 7 and 8,
**characterized in that** the sensor component (20) has an elliptical cross-section, wherein a flat side of the sensor component (20) is arranged in the region of the recess (11).

10. Apparatus according to claim 8,
**characterized in that** the sensor component (20) is a measurement membrane having a cross-section comprising at least two opposite-lying membrane sides (21 ;22) which each are kinked inwards, while a membrane top side (23) which connects the two membrane sides (21 ;22) to each other is formed straight and is arranged in the region of the recess (11).

11. Apparatus according to one of the preceding claims,
**characterized in that** the element (10; 40) comprises a planar flange (40) and a valve plate (41) having a recess (43), wherein the fluid flows at least partially along the flange (40) through the recess (43), and the sensor component (20) comprises a spiral shaped labyrinth (42) opposite the recess (43) for inducing an initial flow.

12. Apparatus according to one of the preceding claims,
**characterized in that** the plunger is surrounded by a hard component comprising an abutment surface underneath the plunger's upper edge, wherein the abutment surface is arranged so that the plunger can only be pressed down by the amount of its overhang and/or to create a preload for a pressure sensor.

13. Apparatus according to one of the preceding claims,
**characterized in that** the sensor component (20) is a membrane, wherein the force coming from the membrane of the sensor component (20) is transferred to the external force sensor (30) in order to recognize an occlusion.

## Revendications

1. Dispositif d'alimentation et de dosage d'un fluide pour applications médicales, comprenant une pompe pour le refoulement du fluide et au moins un composant (10 ; 40) à travers lequel le fluide est refoulé,
dans le composant (10 ; 40), au moins un évidement (11) étant prévu, qui est recouvert de manière étanche par un composant de capteur (20), qui est constitué d'un matériau sensible à la pression, le matériau du composant (10 ; 40) étant plus dur que celui du composant de capteur (20), le dispositif comprenant un capteur de force (30), avec lequel des modifications, induites par la pression, du composant de capteur (20) au niveau de l'évidement (11) sont mesurables, **caractérisé en ce que** le capteur de force (30) comprend un poinçon qui est en contact direct, au niveau de l'évidement (11), avec la surface du composant de capteur (20), afin de détecter une courbure du composant de capteur (20) vers l'extérieur et de la transmettre au capteur de force (30).

2. Dispositif selon la revendication 1,
**caractérisé en ce que** le composant est un manchon tubulaire (10) à travers lequel le fluide est refoulé en direction de la pompe ou loin de la pompe.

3. Dispositif selon l'une ou les deux revendications 1 et 2,
**caractérisé en ce que** le composant est une bride (40) sur laquelle sont montés le manchon tubulaire et/ou un cylindre pour le refoulement du fluide, le fluide s'écoulant au moins partiellement le long de la bride (40).

4. Dispositif selon l'une ou plusieurs des revendications 1 à 3,
**caractérisé en ce que** le composant de capteur (20) est constitué d'un élastomère.

5. Dispositif selon la revendication 4,
**caractérisé en ce que** le composant de capteur (20) est constitué de silicone ou d'un élastomère thermoélastique.

6. Dispositif selon l'une ou plusieurs des revendications 1 à 5,
**caractérisé en ce que** la liaison entre le composant de capteur (20) et le composant (10 ; 40) est Une liaison moulée par injection qui a été établie par un procédé à deux composants.

7. Dispositif selon l'une ou plusieurs des revendications 2 à 6,
**caractérisé en ce que** le composant de capteur (20) est une gaine qui entoure un manchon tubulaire (10) avec une complémentarité de forme de façon à recouvrir l'évidement (11) de manière étanche de l'extérieur.

8. Dispositif selon l'une ou plusieurs des revendications 2 à 6,
**caractérisé en ce que** le composant de capteur (20) est une gaine qui est montée par complémentarité de forme à l'intérieur d'un manchon tubulaire (10), de façon à recouvrir l'évidement (11) de manière étanche de l'intérieur.

9. Dispositif selon l'une ou les deux revendications 7 et 8,
**caractérisé en ce que** le composant de capteur (20) présente une section de forme elliptique, un côté plat du composant de capteur (20) étant disposé au niveau de l'évidement (11).

10. Dispositif selon la revendication 8,
**caractérisé en ce que** le composant de capteur (20) est une membrane de mesure avec une section qui comprend au moins deux côtés de membrane (21 ; 22) superposés, qui sont chacun pliés vers l'intérieur, pendant qu'un côté supérieur de la membrane (23), qui relie entre eux les deux côtés de la membrane (21 ; 22), présente une forme droite et est disposée au niveau de l'évidement (11).

11. Dispositif selon l'une des revendications précédentes,
**caractérisé en ce que** le composant (10 ; 40) comprend une bride plate (40) et un disque de vanne (41) avec un creux (43), le fluide s'écoulant au moins partiellement le long de la bride (40) à travers le creux (43) et le composant de capteur (20) comprenant, en face du creux (43), un labyrinthe en spirale (42) pour l'induction d'un écoulement initial.

12. Dispositif selon l'une des revendications précédentes, **caractérisé en ce que** le poinçon est entouré par un composant dur qui comprend, en dessous d'une arête supérieure du poinçon, une surface de butée, la surface de butée étant conçue afin que le poinçon ne puisse être comprimé que du montant de son porte-à-faux et/ou afin de produire une précontrainte pour des capteurs de pression.

13. Dispositif selon l'une des revendications précédentes, **caractérisé en ce que** le composant de capteur (20) est une membrane, la force exercée par la membrane du composant de capteur (20) étant transmise au capteur de force externe (30) afin de détecter une occlusion.
